# EUROPÄISCHE PATENTSCHRIFT

(11) **EP 2 489 670 B1**
(45) Veröffentlichungstag und Bekanntmachung des Hinweises auf die Patenterteilung: **25.10.2017**
(21) Anmeldenummer: 11006893.9
(22) Anmeldetag: 09.11.2011
(51) Int. Cl.: C07C 227/16, C07C 229/76, C07F 1/08, C07F 3/06, C07F 13/00, C07F 15/02, C07F 15/04

(54) **Verfahren zur Herstellung von Aminosäure-Chelat-Verbindungen, Aminosäure-Chelat-Verbindungen und Verwendung von Aminosäure-Chelat-Verbindungen**
Method for producing amino acid-chelate compounds, amino acid-chelate compounds and use of amino acid-chelate compounds
Procédé de fabrication de composés d'acide aminé-chélate, composés d'acide aminé-chélate et utilisation de composés d'acides aminés-chélate

(30) Priorität: 17.02.2011 DE 102011011924
(43) Veröffentlichungstag der Anmeldung: 22.08.2012
(73) Patentinhaber: ISF GmbH, 25421 Pinneberg (DE)
(72) Erfinder: Ramhold, Dietmar, 23820 Pronstorf (DE); Gock, Eberhard, 38640 Goslar (DE); Mathies, Edmund, 25436 Moorrege (DE); Strauch, Wolfram, 22587 Hamburg (DE)
(74) Vertreter: Hauck Patentanwaltspartnerschaft mbB

(56) Entgegenhaltungen:
- EP-A1- 1 529 775
- DE-A1-102004 036 486

## Beschreibung

Die Erfindung bezieht sich auf ein Verfahren zur Herstellung von Aminosäure-Chelat-Verbindungen.

Wenn Metallverbindungen mit Aminosäuren zur Reaktion gebracht werden, entstehen sogenannte Chelate. Chelatverbindungen gibt es u.a. für die Metalle Kupfer, Zink, Mangan, Eisen, Kalzium, Magnesium, Cobalt, Vanadium, Selen und Nickel und für die Aminosäuren Glycin, Lysin und Methionin.

Aminosäure-Chelat-Verbindungen werden unter anderem in der Tierfütterung und in der Düngung zur Versorgung mit Spurenelementen verwendet. In der Tierernährung werden seit einigen Jahren zunehmend Glycin-Chelate eingesetzt. In vielen Tierversuchen wurden eine Leistungssteigerung und eine verbesserte intestinale Absorption gegenüber Spurenelementen aus anorganischen Verbindungen beobachtet. Die Effizienz von Spurenelementen im Futter lässt sich verbessern und die Ausscheidungsrate reduzieren. Das Risiko einer physiologischen Unterversorgung und Leistungsdepression wird verringert. Darüber hinaus wurden Hinweise über mögliche Vorteile von organisch gebundenen Spurenelementen publiziert, z.B. verbesserte zootechnische und reproduktive Leistungen, höhere äußere und innere Ei-Qualität, höhere Inkorporation in Körperorgane oder Gewebe.

Futtermittelrechtlich zugelassen und am Markt erhältlich sind derzeit folgende Glycin-Chelate (in Klammern sind jeweils die E-Nummern entsprechend der EU-Zusatzstoff-VO angegeben):
- Glycin-Eisenchelat-Hydrat (E1), kurz: Eisen-Glycinat
- Glycin-Kupferchelat-Hydrat (E4), kurz: Kupfer-Glycinat
- Glycin-Manganchelat-Hydrat (E5), kurz: Mangan-Glycinat
- Glycin-Zinkchelat-Hydrat (E6), kurz: Zink-Glycinat.

Die derzeit im Markt erhältlichen Glycinate unterscheiden sich deutlich insbesondere hinsichtlich des Spurenelement-Gehalts, des Glycin-Gehalts, der Löslichkeit in Wasser, der Farbe und Struktur, des pH-Wertes und der Menge und Art der anorganischen Anionen (Sulfate und Chloride). Alle bisher am Markt befindlichen, sogenannten Kupferglycinate sind entweder mit Anionen versetzt und/oder mit Füllstoffen verdünnt und/oder enthalten einen zu geringen Glycingehalt für eine echte zweifache Komplexierung. Die enormen Unterschiede sind auf die jeweils angewendeten Herstellungsverfahren, die eingesetzten Rohstoffe und die gewählten Reaktionsverhältnisse zwischen Spurenelement und Glycin zurückzuführen.

Die Herstellung von Glycin-Chelaten ist äußerst aufwendig. Sie geht im Allgemeinen von Lösungen der entsprechenden Spurenelementverbindungen mit Glycin aus, die bei erhöhten Temperaturen zur Reaktion gebracht werden. Es schließen sich Eindampfen, Kristallisation, Trocknung und Mahlung an.

Der Stand der Technik ist beispielsweise in der US 4,315,927A, US 4,814,177A, US 830,716A, US 4,599,152A sowie US 5,516,925A beschrieben.

Die Patentanmeldung CN 2009/10030766.3 beschreibt die Herstellung von Zink-Glycinat. Danach werden im ersten Schritt 5 bis 15% Glycin mit 5-10%igem Nano-ZnO mit Wasser bei 50°-80°C 3 bis 24 Stunden gerührt und anschließend 6 bis 10 Stunden ruhend gehalten. Im zweiten Schritt wird bei 3.000 bis 8.000 min⁻¹ zentrifugiert und das Zentrifugat bei 80°-120°C im Ofen getrocknet. Der dritte Schritt umfasst die Zerkleinerung und die Klassierung bei größer als 80-120 mesh. In der Anmeldung CN 2009/10030767.8 wird das gleiche Herstellungsverfahren für Calciumglycinat unter Verzicht auf das Zentrifugieren beschrieben.

Die EP 1 529 775 B1 betrifft ein Verfahren zur Herstellung von Chelaten von Metallen mit organischen Säuren, das im Wesentlichen im wasserfreien Medium arbeitet. Eingesetzt werden Metalloxide, Hydroxide oder Salze. Der organische Säureligant wie z.B. Glycin, Lysin, Glutaminsäure u.a. und die jeweiligen Metallverbindungen wie z.B. Hydroxide wie Kupferhydroxid, Zinkhydroxid, Eisenhydroxid, Manganhydroxid usw. werden in nichtwässrigen Flüssigkeiten wie Methanol, Ethanol, i-Propanol, Hexan, Petroleumether usw. eingetaucht und bei Raumtemperatur oder erhöhter Temperatur miteinander vermischt. Da Wasser auch ein Reaktionsprodukt ist, muss es mit Hilfe einer Wasserabtrennvorrichtung (z.B. Dean-Stark-Wasserabscheider) entfernt werden. Die Entfernung des jeweiligen Metallchelats aus der organischen Flüssigkeit erfolgt durch Filtrieren. Nach dem Trocknen stellt das jeweilige Metallchelat ein sehr feines Pulver als Fertigprodukt dar.

Aus dem Ausführungsbeispiel der EP 1 529 775 B1 geht hervor, dass der beschriebene Herstellungsprozess einer Vorbehandlung der eingesetzten Metallverbindungen bedarf. So wird z.B. für die Herstellung von Kupferhydroxid von CuSO₄ · 5H₂O ausgegangen, das mit KOH bei pH 10-11 zur Ausfällung von Cu(OH)₂ stabilisiert wird. Es schließt sich eine zweimalige Zentrifugierung an, die durch Waschvorgänge in Ethanol begleitet werden. Zur Herstellung von Kupferglycinat wird dann Cu(OH)₂ mit Glycin gemischt und diese Mischung über fünf Stunden in Ethanol gekocht. Das unter diesen Bedingungen erzeugte Kupferglycinat wird abfiltriert und zu Pulver getrocknet.

Die Patentanmeldungen CN 92107282.1 und CN 2007/130121.0 beschreiben die Umsetzung von Gemischen aus Kupferacetat und Glycin in einer Einschritt-Festkörperreaktion in einer Kugelmühle. Dazu wird eine Mischung von Kupferacetat und Glycin mit Wasser und Natriumcarbonat versetzt und einer Nassmahlung in einer Kugelmühle unterzogen. Nach mehrstündiger Mahlung wird die Suspension getrocknet, mit Ethanol gewaschen, zentrifugiert und nochmals getrocknet.

Die DE 10 2004 039 486 A1 beschreibt ein trockenes Verfahren zur Herstellung organischer Spurenelementverbindungen. Ein beliebiges trockenes Gemisch aus einem Metalloxid und einer festen organischen Säure wird einer mechanischen Beanspruchung durch Schlag und Druck einer Feinstzerkleinerungsmaschine derart ausgesetzt, dass der freigesetzte Enthalpiebetrag eine Festkörperreaktion zu einer metallsalzartigen Verbindung auslöst.

Im Mittelpunkt dieser Offenlegungsschrift steht die Herstellung von Zinkbismethionat aus Gemischen von ZnO und Methionin, die im Gemisch miteinander vermahlen werden. Dies wird mit insgesamt sieben Beispielen belegt. Die übrigen drei Beispiele haben Gemische aus CuO und Asparaginsäure (eine von insgesamt 21 Aminosäuren), Gemische aus MnO und Apfelsäure (Carbonsäureesther) sowie Gemische aus Cr(OH)₃ und Nikotinsäure (Alkaloide salzartig an Pflanzensäure gebunden) zum Gegenstand.

Die Überprüfung des Verfahrens hat ergeben, dass Betriebsstörungen durch Anbacken von Mahlgut an den Mühlenwänden auftreten können. Diese Anbackungen können die vollständige Zementierung des Mahlraumes hervorrufen, was sich nur mit Hilfe von Drucklufthämmern wieder beheben lässt und eine industrielle Anwendung ausschließt. Zudem sind die Produktqualitäten nicht reproduzierbar.

Davon ausgehend liegt der Erfindung die Aufgabe zugrunde, ein einfaches, stabiles und industrietaugliches Verfahren für die Herstellung von Aminosäure-Chelat-Verbindungen zur Verfügung zu stellen.

Die Aufgabe wird durch ein Verfahren mit den Merkmalen des Anspruchs 1 gelöst. Vorteilhafte Ausgestaltungen des Verfahrens sind in Unteransprüchen angegeben.

Bei dem erfindungsgemäßen Verfahren zur Herstellung von Aminosäure-Chelat-verbindungen werden Metalloxide und/oder Metallcarbonate und/oder Metallsulfate und/oder Metallchloride und/oder Metallhydroxide in fester Form mechanisch aktiviert und danach die aktivierten Metalloxide und/oder Metallcarbonate und/oder Metallsulfate und/oder Metallchloride und/oder Metallhydroxide mit Aminosäuren in fester Form zusammengebracht und in einer Festkörperreaktion zu Aminosäure-Chelat-Verbindungen umgesetzt, wobei die Aktivierung und/oder die Umsetzung in einer Exzenter-Schwingmühle und/oder in einer anderen Schwingmühle und/oder in einer Rührwerkskugelmühle und/oder in einer Trommelmühle und/oder in einem anderen Mischreaktor durchgeführt werden und/oder bei dem die Aktivierung und/oder die Umsetzung durch mechanische Beanspruchung durch Schlag und Druck in einer Feinzerkleinerungsmaschine erfolgt.

Es wurde gefunden, dass die mechanische Aktivierung von Gemischen aus Metallverbindungen und Aminosäuren kein geeigneter Weg ist. Der Umsatz von Metallverbindungen mit der organischen Säure ist vielmehr eine spontane kettenartige Folgereaktion. Aus energetischer Sicht ergibt sich bei der mechanischen Aktivierung von Gemischen, deren größerer Masseanteil keiner mechanischen Aktivierung bedarf, daher ein Energieverlust großen Ausmaßes. Dieser liegt in der Größenordnung des Mischungsverhältnisses. Bei einem Mischungsverhältnis von 1:2 (CuO zu Glycin) beträgt der nicht mechanisch zu aktivierende Anteil mehr als 50%. Der entscheidende Reaktant beim Umsatz mit organischen Säuren ist die Metallverbindung. Erfindungsgemäß wird diese zunächst mechanisch aktiviert. Die Entkopplung der mechanischen Aktivierung der Metallverbindung und der Festkörperreaktion mit einer Aminosäure führt zu einer entscheidenden Einflussnahme auf den Reaktionsmechanismus bei der Synthese von Metall-Chelaten. Nach der separaten mechanischen Aktivierung der Metallverbindung wird die Chelat-Festkörperreaktion durch Zusetzen von Aminosäuren ausgelöst. Der Gesamtumsatz der Reaktion wird durch die gesonderte mechanische Aktivierung der Metallverbindung beträchtlich verbessert. Bei der Festkörperreaktion liegen die Metallverbindung und die Aminosäure in fester Form vor. Hierbei sind die Reaktionspartner trocken oder im Wesentlichen trocken, d.h. weisen allenfalls geringe Feuchtegehalte auf. Ihre Feuchteanteile betragen bevorzugt höchstens 5 Gew.-%.

Gemäß einer bevorzugten Ausgestaltung werden der mechanischen Aktivierung die Metallverbindungen als Gemenge aus losen Partikeln zugeführt. Gemäß einer weiteren Ausgestaltung werden die Aminosäuren den mechanisch aktivierten Metallverbindungen in Form eines weiteren Gemenges aus losen Partikeln zugeführt. Die mechanische Aktivierung bzw. die Festkörperreaktion werden durch den Einsatz der Metallverbindungen in Form eines Gemenges aus losen Partikeln bzw. der Aminosäuren in Form eines Gemenges aus losen Partikeln begünstigt. Grundsätzlich ist es aber auch möglich, die Reaktionspartner dem Verfahren in Form großer zusammenhängender Stücke zuzuführen, die bei der Durchführung des Verfahrens zerkleinert werden können.

Gemäß einer Ausgestaltung des Verfahrens wird mindestens ein Reaktionsteilnehmer thermisch aktiviert. Die thermische Aktivierung beschleunigt die Festkörperreaktion. Bei der thermischen Aktivierung wird mindestens ein Reaktionsteilnehmer erwärmt. Überschreitet die Reaktionstemperatur den Siedepunkt des Wassers (100°C bei Durchführung der Reaktion unter Normaldruck), so verdampft das freie Reaktionswasser und wird von den Reaktionsteilnehmern getrennt.

Gemäß einer bevorzugten Ausgestaltung erfolgt die thermische Aktivierung gleichzeitig mit der mechanischen Aktivierung und/oder mit der Umsetzung in der Festkörperreaktion. Hierbei wird die für die thermische Aktivierung erforderliche Wärmeenergie der Festkörperreaktion gezielt zugeführt. Ferner kann die bei der mechanischen Aktivierung bzw. bei der Durchführung der Festkörperreaktion freigesetzte Wärmeenergie für die thermische Aktivierung genutzt werden. Dies ist insbesondere bei Durchführung der mechanischen Aktivierung und/oder der Festkörperreaktion in einer Mühle oder einem anderen Mischreaktor der Fall.

Gemäß einer weiteren Ausgestaltung wird bei der Umsetzung entstehendes Wasser von den Reaktionsteilnehmern getrennt. Hierdurch wird ein Anbacken von Reaktionsteilnehmern an festen Oberflächen und die damit verbundene Beeinträchtigung der Reaktion vermieden. Die mit dem Anbacken im Reaktor verbundenen Betriebsstörungen und Instandsetzungsarbeiten werden ebenfalls vermieden. Dies gilt insbesondere bei Durchführung der Festkörperreaktion in einer Mühle oder einem anderen Mischreaktor.

Das Wasser kann insbesondere durch Verdampfen von den Aminosäure-Chelaten abgetrennt werden. Hierbei kann Wärme zugeführt und/oder der Druck reduziert werden, unter dem die Festkörperreaktion durchgeführt wird. Durch Wärmezufuhr kann zugleich die thermische Aktivierung erfolgen. Ferner kann die Festkörperreaktion in Gegenwart absorbierender Feststoffe durchgeführt werden.

Gemäß einer weiteren Ausgestaltung werden die Ausgangsstoffe dem Verfahren trocken zugeführt. Hierdurch wird das Risiko des Anbackens an festen Oberflächen weiter vermieden. Dafür haben die Ausgangsstoffe bevorzugt einen Wasseranteil von maximal 5%. Weiterhin bevorzugt beträgt der Wasseranteil höchstens 2,5%.

Gemäß einer weiteren Ausgestaltung werden die Aktivierung und die Umsetzung in demselben Reaktor durchgeführt. In einen diskontinuierlichen Reaktor können zunächst die Metallverbindungen eingegeben und aktiviert werden und danach die Aminosäuren zugegeben und die Festkörperreaktion durchgeführt werden. In einem kontinuierlichen Durchlaufreaktor können an einer ersten Zuführposition die Metallverbindungen zugeführt werden und nach Durchlaufen einer Aktivierungsstrecke an einer zweiten Zuführposition die Aminosäuren zugeführt werden, um gemeinsam mit den aktivierten Metallverbindungen eine Reaktionsstrecke zu durchlaufen.

Gemäß einer anderen Ausgestaltung werden die Aktivierung und die Umsetzung in verschiedenen Reaktoren durchgeführt. Die verschiedenen Reaktoren können diskontinuierliche Reaktoren sein, in denen die mechanische Aktivierung und die Umsetzung getrennt und absatzweise durchgeführt werden. Ferner können die verschiedenen Reaktoren kontinuierliche Reaktoren sein, in denen die mechanische Aktivierung und die Umsetzung von einander getrennt im Durchlauf durchgeführt werden.
Für die Aktivierung und wahlweise die Festkörperreaktion werden die Metallverbindungen und wahlweise die Aminosäure bevorzugt einer mechanischen Beanspruchung durch Schlag und Druck in einer Feinzerkleinerungsmaschine ausgesetzt. Diese ist bevorzugt eine Exzenter-Schwingmühle.

In einer Exzenter-Schwingmühle wird das behandelte Gut einer mechanischen Beanspruchung vornehmlich durch Schlag und Druck ausgesetzt. Eine Exzenter-Schwingmühle ermöglicht derzeit die effektivste mechanische Aktivierung von Metallverbindungen und ist auch für die Durchführung der Festkörperreaktion sehr gut geeignet. Gleichzeitig kann von der in der Exzenter-Schwingmühle freigesetzten Wärmeenergie die thermische Aktivierung bewirkt werden.

Eine für den Einsatz in dem erfindungsgemäßen Verfahren geeignete Exzenter-Schwingmühle ist in der DE 43 35 797 C2 beschrieben. Geeignete Schwingmühlen werden von der Firma Siebtechnik, Mülheim an der Ruhr, Deutschland, vertrieben.

Die Aktivierung und die Umsetzung können in demselben Mischreaktor, in verschiedenen Mischreaktoren desselben Typs oder in verschiedenen Mischreaktoren unterschiedlichen Typs durchgeführt werden. Insbesondere kann die Aktivierung in einer Exzenter-Schwingmühle und die Umsetzung in einem anderen Typ Mischreaktor durchgeführt werden.

Gemäß einer Ausgestaltung wird die durch das Betreiben des Mischreaktors erzeugte Wärme zur thermischen Aktivierung und/oder Verdampfung des Wassers genutzt. Insbesondere kann die durch Betrieb einer Exzenter-Schwingmühle erzeugte Wärme allein die thermische Aktivierung und/oder Verdampfung des Wassers bewirken. Zum Aufheizen des Mischreaktors wird dieser ggfs. zunächst in einer Aufwärmphase aufgeheizt. Die Aufwärmphase kann mit der mechanischen Aktivierung zusammenfallen.

Gemäß einer Ausgestaltung wird dem Reaktor die Wärme für die thermische Aktivierung und/oder für das Verdampfen von Wasser zugeführt. Die thermische Aktivierung und/oder die zur Verdampfung erforderliche Wärme kann dem Reaktor ausschließlich von außen zugeführt werden. Ggfs. kann zusätzlich zu der vom Reaktor erzeugten Wärme weitere Wärme von außen zugeführt werden.

Gemäß einer weiteren Ausgestaltung wird die thermische Aktivierung und/oder Verdampfung bei einer Temperatur zwischen 30° und 150°C durchgeführt. Weiterhin wird die thermische Aktivierung und/oder die Verdampfung bei einer Temperatur von 80° bis 120°C durchgeführt.

Gemäß einer weiteren Ausgestaltung wird bei der Umsetzung entstehendes Wasser aus dem Reaktor abgeführt. Das Wasser kann bei der Umsetzung einmalig, intermittierend oder kontinuierlich abgeführt werden.

Gemäß einer weiteren Ausgestaltung wird die Umsetzung während einer Lagerung des Reaktionsproduktes außerhalb des Reaktors fortgesetzt. Die Lagerung des Reaktionsproduktes vor der Anwendung kann für die Weiterführung der Reaktion genutzt werden. Hierdurch wird die Verfügbarkeit des Reaktors für die Aktivierung erhöht.

Gemäß einer bevorzugten Ausgestaltung enthält das Reaktionsprodukt für eine Fortsetzung der Umsetzung während einer Lagerung bei Entnahme aus dem Reaktor freies Reaktionswasser. Bei einer Lagerung bei Temperaturen unterhalb des Siedepunktes, insbesondere bei Raumtemperatur, begünstigt das freie Reaktionswasser durch Ionentransport eine Fortsetzung der Reaktion. Somit kann die Reaktion während einer Lagerung fortgesetzt werden. Während der Umsetzung im Reaktor kann die Trennung des Wassers von den Reaktionsteilnehmern so gesteuert werden, dass ein Anbacken an festen Oberflächen vermieden wird und ein hinreichender Anteil freien Reaktionswassers für eine Umsetzung während einer nachfolgenden Lagerung in dem Reaktionsprodukt verbleibt.

Gemäß einer weiteren Ausgestaltung beträgt der Gehalt des freien Wassers im Produkt zwischen 1% und 5%. Bevorzugt beträgt der Wassergehalt des Produktes maximal 3%. Weiterhin bevorzugt beträgt er etwa 2,5%. Bei diesen Wasseranteilen wird vermieden, dass das Produkt verklumpt und die Weiterverarbeitung beeinträchtigt wird. Die Abtrennung des Wassers bei der Umsetzung bzw. während einer nachfolgenden Lagerung kann so gesteuert werden, dass der Wassergehalt des Produktes entsprechend reduziert wird.

Gemäß einer weiteren Ausgestaltung erfolgt die Umsetzung bis zur vollständigen Stöchiometrie. Doppelt komplexiertes Kupfer-Glycinat besteht aus 29,7 Gew.-% Kupfer und 70,3 Gew.-% Glycin. Das Massenverhältnis von Kupfer zu Glycin ist somit 1:2,37. Bei der mechanischen Aktivierung von CuO wird eine, unabhängig von der Glycinatreaktion erhöhte Wasserlöslichkeit erreicht. Dadurch kann der lösliche Kupfergehalt von Kupferglycinat überstöchiometrisch sein und mehr als 35% erreichen.

Gemäß einer weiteren Ausgestaltung werden die Aminosäuren der Umsetzung überstöchiometrisch zugeführt. Hierdurch wird eine Umsetzung bis zur vollständigen Stöchiometrie begünstigt.

Gemäß einer weiteren Ausgestaltung beträgt das Massenverhältnis der Metalloxide und/oder der Metallcarbonate und/oder der Metallsulfate und/oder Metallchloride und/oder Metallhydroxide zu den Aminosäuren 1:2 bis 1:5.

Gemäß einer Ausgestaltung werden Aminosäure-Chelat-Verbindungen des Kupfers und/oder des Zinks und/oder des Mangans und/oder des Eisens und/oder des Magnesiums und/oder des Kalziums und/oder des Nickels und/oder des Cobalts hergestellt. Gemäß einer weiteren Ausgestaltung werden Aminosäure-Chelat-Verbindungen des Glycins und/oder des Lysins und/oder des Methionins und/oder von weiteren Aminosäuren und/oder von Aminosäuregemischen hergestellt.

Für komplexe Anwendungen, z. B. als Gärzusatz und als Düngemittelzusatz, erlaubt das erfindungsgemäße Verfahren die Herstellung von Metallkombi-Chelatverbindungen, in dem Gemenge von Metallverbindungen z. B. des Kupfers, Zinks, Eisens und Mangans mechanisch aktiviert werden und z. B. mit Glycin durch thermische Aktivierung zur Reaktion gebracht werden.

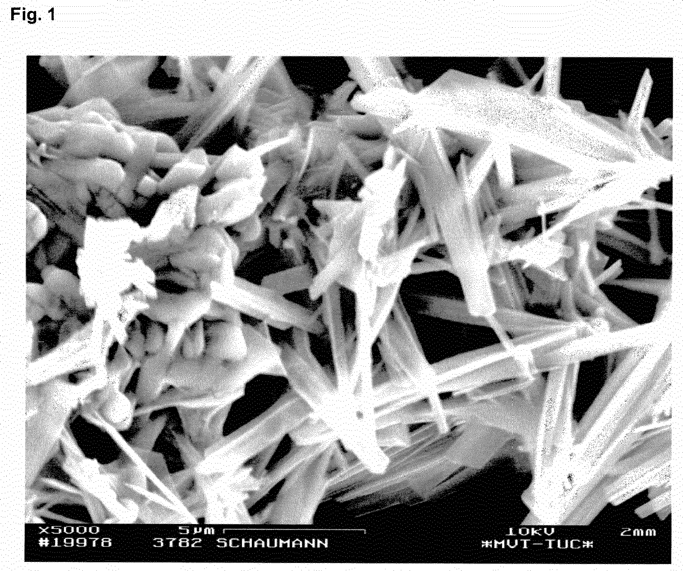

Erfindungsgemäß hergestellte Aminosäure-Chelat-Verbindungen weisen Partikel mit einer charakteristischen nadelförmigen Kristallstruktur auf. Diese Struktur ist unter dem Rasterelektronenmikroskop sichtbar, Abb. 1 zeigt beispielhaft eine Aufnahme mit dem Rasterelektronenmikroskop (REM).

Gemäß einer Ausführungsart enthalten die Aminosäure-Chelat-Verbindungen keine Sulfate und/oder keine Chloride und weisen einen pH-Wert im Bereich von 4-9 auf. Für den Einsatz als Futtermittelzusatz, Nahrungsmittelzusatz, Nahrungsergänzungsmittel und Galvanikzusatz hat dies den Vorteil, dass keine unerwünschten Anionen eingetragen werden.

Gemäß einer Ausführungsart weisen die Aminosäure-Chelate eine mittlere Partikelgröße von 40 bis 60 µm, vorzugsweise etwa 50 µm auf, und liegen bis zu 80% der Partikel im Bereich von 0 - 100 µm und sind bis zu 2% größer als 500 µm. Diese Körnung ist besonders vorteilhaft für die Verwendung als Futtermittelzusatz, Nahrungsmittelzusatz und Nahrungsergänzungsmittel, weil eine gute Verteilbarkeit und Mischgüte auch bei geringen Konzentrationen gegeben ist.

Gemäß einer Ausführungsart sind die Aminosäure-Chelat-Verbindungen nach dem Verfahren der oben beschriebenen Art herstellbar.

Die erfindungsgemäß hergestellten Aminosäure-Chelat-Verbindungen können als Futtermittelzusatz und/oder als Gärzusatz und/oder als Düngemittelzusatz und/oder als Nahrungsmittelzusatz und/oder als Nahrungsergänzungsmittel und/oder als Galvanikzusatz verwendet werden.

Die Funktionsfähigkeit des erfindungsgemäßen Verfahrens wird beispielsweise am System CuO-Glycin unter stöchiometrischen Bedingungen dargestellt.

Die trockene Synthese lässt sich mit folgendem Mechanismus beschreiben:

### 1. Schritt

Separate mechanische Aktivierung von CuO

### 2. Schritt

Zusatz von Glycin.

Thermische Aktivierung und temperaturbestimmter Umsatzgrad mit mechanisch aktiviertem CuO.

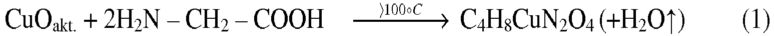

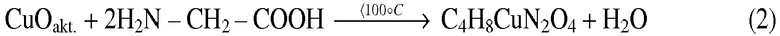

Danach erfolgt im ersten Schritt die separate mechanische Aktivierung von CuO in einer Exzenter-Schwingmühle mit z.B. einem Energieaufwand von ca. 300 kWh/t, bei einem Zeitbedarf von nur ca. 30 bis 60 min. Die Mühlenfüllung beträgt 30%. Da bei Mahlprozessen ca. 90% der aufgebrachten Energie in Wärme umgewandelt werden, ergeben sich bei nicht thermostatisierter Mühle Temperaturen zwischen 30 und 150°C.

Nach Beendigung der mechanischen Aktivierung von CuO wird die Mühlenfüllung im zweiten Schritt durch Zusatz von Glycin auf 100% erhöht.

Mit einem Energieaufwand von nur ca. 5 kWh/t werden CuO und Glycin in wenigen Minuten zur Reaktion gebracht. Die Geschwindigkeit der Reaktion ist abhängig von der Betriebstemperatur der Mühle, die die thermische Aktivierung hervorruft. Der Grad der thermischen Aktivierung wird von der Höhe der Betriebstemperatur bestimmt. Diese Vorgehensweise gilt bei Batch-Betrieb in einer Mühle.

Es ist jedoch auch möglich, die mechanische und die thermische Aktivierung durch eine Reihenschaltung zu trennen. In diesem Fall würde die entkoppelte thermische Aktivierung in einer zweiten thermostatischen Schwingmühle oder einer thermostatisierten herkömmlichen Trommelmühle bzw. einer thermostatisierten Rührwerkskugelmühle oder in einem thermostatisierten Mischer durchgeführt werden können.

Bei Temperaturen größer 100°C liegt der Umsatz z.B. zwischen 95 und 100%. Unterhalb 100°C ermöglicht das verbleibende Reaktionswasser die Fortsetzung der Reaktion durch Lagerung bis zum vollständigen stöchiometrischen Umsatz.

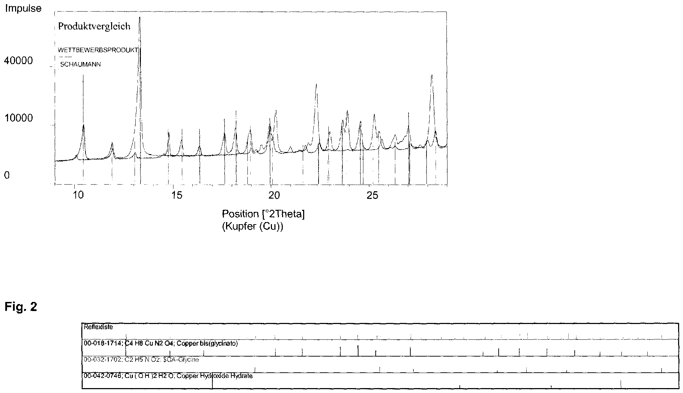

Als Kontrolle für den Umsatzgrad sind Lösetests mit Wasser sehr zuverlässig, wobei die blaue Farbe des Tetraminkomplexes sichtbar wird. Durch Röntgenfeinstrukturaufnahmen lässt sich weiterhin belegen, dass es sich bei der entstandenen Verbindung nach der gültigen ASTM-Kartei um Copperbis (Glycinato) entsprechend Glycin-Kupfer-Chelat handelt. Abb. 2 zeigt beispielhaft ein Röntgenbeugungsdiagramm. Die Röntgenbeugung beruht auf der Bestrahlung von Pulverproben mit monochomatischem Röntgenlicht, Gemessen wird die reflektierte Strahlungsintensität in Abhängigkeit vom Beugungswinkel. Dabei ergeben sich Intensitätsmaxima bei definierten Winkeln (20), bei denen bestimmte Kristallflächen, in diesem Fall Kristallflächen des Kupfer-Glycinats, die Röntgenstrahlen reflektieren. Durch Vergleich mit reinsten Referenzproben des Kupfer-Glycinats mit definierten Beugungsmustern ist eine eindeutige Zuordnung von kristallinen Stoffen möglich.

Der Gegenstand der Erfindung wurde auf die trockene Synthese von ZinkChelat, Mangan-Chelat, Eisen-Chelat, Nickel-Chelat sowie Magnesium-Chelat, Calcium-Chelat und Cobalt-Chelat ausgedehnt und der Reaktionsmechanismus in gleicher Weise bestätigt.

Nachfolgend wird das erfindungsgemäße Verfahren anhand von Beispielen belegt.

### Beispiel 1:

Die Versuche für das erfindungsgemäße Verfahren wurden in einem Satellitenmahlbehälter mit einem Volumen von 2,7 1 durchgeführt, der an eine Exzenter-Schwingmühle vom Typ 656-0,5 ks angeflanscht wurde. Der Mahlraum des Satelliten war mit Keramik ausgekleidet, um Kontaminationen zu vermeiden.

Es wurde bei folgenden Betriebsbedingungen gearbeitet:

| | |
|---|---|
| Drehzahl: | 960 min⁻¹ |
| Amplitude: | 12 mm |
| Mahlkörper: | Stahl |

150 g eines Kupferoxid-Pulvers mit einer Korngröße von < 100 µm wurden 15 Minuten im oben genannten Satelliten einer mechanischen Aktivierung unterzogen. Zu Beginn der Aktivierung hatte die Mühle 30 °C. Danach wurde die Mühle angehalten und zusätzlich 350 g Glycin dem aktivierten Kupferoxid zugeführt. Die Betriebstemperatur der Mühle betrug dann 130 °C. Nach 10minütiger thermischer Aktivierung des Gemenges wurde der Prozess beendet und Wasserdampf über ein Ablassventil entspannt. Bereits die leicht bläuliche Farbe des Produktes zeigte an, dass eine Festkörperreaktion abgelaufen sein musste und dass es sich um einen neuen Stoff handelte. Das Produkt wurde analysiert auf Wasserlöslichkeit, Kristallgitterstruktur, Kornform und Korngröße. Die Wasserlöslichkeit ergab bei Raumtemperatur nach 10 min. 58%, und nach 60 min. 98% dabei wird das gelöste Cu in Gegenwart von NH₃-Ionen in den bekannten blauen Tetraminkomplex [Cu(NH₃)₄]⁻² überführt. Die Überprüfung der Kristallstruktur mit dem Röntgendiffraktometer X'Pert der Fa. Philips zeigte beim Röntgenbeugungswinkel 20 10,3 den Hauptpeak von Copperbisglycinato (C₄H₈CuN₂O₄), das in der ESTM-Kartei unter der Nummer 00-018-1714 ausgewiesen ist.

Das Ergebnis der Messung des Röntgendiffraktometers ist in Abb. 2 gezeigt.

Die Kornform wurde mit dem Rasterelektronenmikroskop überprüft und ergab die typischen nadelförmigen Kristalle der Glycinate und deren Agglomerate. Sie ist in Abb. 1 gezeigt. Im Hinblick auf die Schüttguteigenschaften fand die Kornverteilungsanalyse einen d₅₀-Wert von 50 µm. Das Material ist rieselfähig, hat einen Wassergehalt < 2% und ist beständig.

### Beispiel 2:

Die Anwendbarkeit des erfindungsgemäßen Verfahrens für die Erzeugung von Kupfer-, Zink-, Eisen-, Mangan- und Nickelglycinaten wird anhand eines Gemenges aus CuO, ZnO, FeSO₄ · H₂O, MnCO₃ und NiO demonstriert. Dazu wurden in der gleichen Versuchseinrichtung wie in Beispiel 1 150 g des oben genannten Gemenges, in dem jede Komponente 20% ausmachte, 25 min. mechanisch aktiviert. Der Satellit wurde anschließend geöffnet und 300 g Glycin zugesetzt. Bei einer Betriebstemperatur von 105 °C wurde die thermische Aktivierung 5 min. lang angeschlossen. Der entstehende Wasserdampf wurde über einen Ablassventil entspannt.

Der Lösetest mit Wasser bei 25 °C ergab nach 60 min. eine vollständige Verfügbarkeit aller eingesetzten Metalle. Die wässrige Lösung war klar und wies eine geringe olive Mischfarbe aus. Auf Röntgenstrukturaufnahmen wurde verzichtet, da die Glycinatlinien der eingesetzten Metalle sich überschneiden.

### Beispiel 3

Als weiteres Beispiel für die Anwendbarkeit des erfindungsgemäßen Verfahrens wird die Herstellung der Erdalkali-Glycinate, Magnesium- und Calcium-Glycinat beschrieben.

Es wurde die gleiche Versuchseinrichtung wie in Beispiel 1 eingesetzt. Die Vorgehensweise für beide Synthesen war identisch, so dass sie hier zusammengefasst werden. 54 g CaO bzw. MgO wurden jeweils einer 10minütigen mechanischer Aktivierung unterzogen. Um für die thermische Aktivierung eine genügend hohe Betriebstemperatur zu haben, war die Mühle bereits vor den Versuchen im Leerlauf auf eine Betriebstemperatur von 110 °C gebracht worden. Nach der mechanischen Aktivierung erfolgte in beiden Fällen eine thermische Aktivierung von jeweils 15 min. Als Lösetest wurde die Wasserlöslichkeit bei jeweils 45 °C über einen Zeitraum von 10 min. getestet.

Bei Eintrag des erzeugten Calcium-Glycinates in Wasser ergab sich eine spontane vollständige Löslichkeit und es stellte sich in der glasklaren Lösung ein pH-Wert von 7,5 ein.

Das erzeugte Magnesium-Glycinat führte beim Einbringen in Wasser zu einem pH-Wert von 8, wobei sich eine leicht trübe Lösung ergab. Die Einstellung von pH 6 mit HCl (Bestandteil der Magensäure) ergab eine klare Lösung ohne die geringsten Spuren von ungelöstem Einsatzstoff. Unter den gleichen Lösebedingungen ist reines MgO in Gegenwart von HCl bis pH 0 stabil.

## Patentansprüche

1. Verfahren zur Herstellung von Aminosäure-Chelat-Verbindungen, **dadurch gekennzeichnet, dass** Metalloxide und/oder Metallcarbonate und/oder Metallsulfate und/oder Metallchloride und/oder Metallhydroxide in fester Form mechanisch aktiviert werden und danach die aktivierten Metalloxide und/oder Metallcarbonate und/oder Metallhydroxide und/oder Metallsulfate und/oder Metallchloride mit Aminosäuren in fester Form zusammengebracht und in einer Festkörperreaktion zu Aminosäure-Chelat-Verbindungen umgesetzt werden, wobei die Aktivierung und/oder die Umsetzung in einer Exzenter-Schwingmühle und/oder in einer anderen Schwingmühle und/oder in einer Rührwerkskugelmühle und/oder in einer Trommelmühle und/oder in einem anderen Mischreaktor durchgeführt werden und/oder bei dem die Aktivierung und/oder die Umsetzung durch mechanische Beanspruchung durch Schlag und Druck in einer Feinzerkleinerungsmaschine erfolgt.

2. Verfahren nach Anspruch 1, bei dem mindestens ein Reaktionsteilnehmer thermisch aktiviert wird.

3. Verfahren Anspruch 1 oder 2, bei dem bei der Umsetzung entstehendes Wasser durch Verdampfen von den Reaktionsteilnehmern getrennt wird.

4. Verfahren nach einem der Ansprüche 1 bis 3, bei dem die Ausgangsstoffe trocken zugeführt werden.

5. Verfahren nach einem der Ansprüche 1 bis 4, dem die Metallverbindungen als Gemenge loser Partikel und/oder Aminosäuren als Gemenge loser Partikel zugeführt werden.

6. Verfahren nach Anspruch 5, bei dem die durch Betreiben des Mischreaktors erzeugte Wärme zur thermischen Aktivierung und/oder zur Verdampfung des Wassers genutzt wird.

7. Verfahren nach einem der Ansprüche 2 bis 6, bei dem die thermische Aktivierung und/oder die Verdampfung bei einer Temperatur zwischen 30 und 150 °C durchgeführt wird, vorzugsweise bei einer Temperatur von 80 bis 120 °C durchgeführt wird.

8. Verfahren nach einem der Ansprüche 1 bis 7, bei dem die Umsetzung während einer Lagerung des Reaktionsproduktes außerhalb eines Reaktors fortgesetzt wird und/oder bei dem das Reaktionsprodukt bei Entnahme aus dem Reaktor freies Reaktionswasser enthält.

9. Verfahren nach einem der Ansprüche 1 bis 8, bei dem Aminosäuren-Chelate des Kupfers und/oder des Zinks und/oder des Mangans und/oder des Eisens und/oder des Magnesiums und/oder des Kalziums und/oder des Nickels und/oder des Cobalts hergestellt werden und/oder bei dem Aminosäuren-Chelate des Glycins und/oder des Lysins und/oder des Methionins und/oder von weiteren Aminosäuren und/oder von Aminosäuregemischen hergestellt werden.

10. Verfahren nach einem der Ansprüche 1 bis 9, bei dem das Massenverhältnis der Metalloxide und/oder der Metallcarbonate und/oder Metallhydroxide und/oder der Metallsulfate und/oder der Metallchloride zu den Aminosäuren 1:2 bis 1:5 beträgt.

## Claims

1. A method for producing amino acid chelate compounds, **characterized in that** metal oxides and/or metal carbonates and/or metal sulfates and/or metal chlorides and/or metal hydroxides in solid form are activated mechanically and then the activated metal oxides and/or metal carbonates and/or metal hydroxides and/or metal sulfates and/or metal chlorides are brought together with amino acids in solid form and converted to amino acid chelate compounds in a solid-state reaction, in which the activation and/or conversion takes place with an eccentric vibrating grinding mill and/or in another vibrating grinding mill and/or with an agitator ball and/or in a drum mill and/or with a mixed reactor and/or the activation and/or conversion takes place through mechanical stress by strike and pressure in a fine crushing machine.

2. The method according to claim 1, in which at least one reactant is thermally activated.

3. The method according to claim 1 or 2, in which the water generated by the conversion is separated from the reactance by evaporation.

4. The method according to one of claims 1 to 3, in which the raw materials are added dry.

5. The method according to one of claims 1 to 4, in which the metal compounds are added as a mixture of loose particles and/or amino acids as a mixture of loose particles.

6. The method according to claim 5, in which the heat generated through operation of the mixed reactor is used for the thermal activation and/or for the evaporation of the water.

7. The method according to one of claims 2 to 6, in which the thermal activation and/or the evaporation is performed at a temperature between 30 and 150 °C, preferably at a temperature of 80 to 120 °C.

8. The method according to one of claim 1 to 7, in which the conversion is continued during storage of the reaction product outside the reactor and/or the reaction product contains free reaction water upon removal from the reactor.

9. The method according to one of claims 1 to 8, in which amino acid chelates of the copper and/or the zinc and/or the manganese and/or the iron and/or the magnesium and/or the calcium and/or the nickel and/or the cobalt are produced and/or in which amino acid chelates of the glycine and/or the lysine and/or the methionine and/or other amino acids and/or amino acid mixtures are produced.

10. The method according to one of claims 1 to 9, in which the mass ratio of the metal oxides and/or the metal carbonates and/or the metal hydroxides and/or the metal sulfates and/or the metal chlorides to amino acids is 1:2 to 1:5.

## Revendications

1. Procédé de fabrication de composés d'acide aminé-chélate, **caractérisé en ce que** des oxydes métalliques et/ou carbonates métalliques et/ou sulfates métalliques et/ou chlorures métalliques et/ou hydroxydes métalliques sont activés mécaniquement sous forme solide et les oxydes métalliques et/ou carbonates métalliques et/ou hydroxydes métalliques et/ou sulfates métalliques et/ou chlorures métalliques activés sont ensuite réunis avec des acides aminés sous forme solide et transformés dans une réaction de corps solides en composés d'acide aminé-chélate, dans lequel l'activation et/ou la transformation sont réalisées dans un broyeur vibrant à excentrique et/ou dans un autre broyeur vibrant et/ou dans un broyeur à billes à agitateur et/ou dans un broyeur à tambour et/ou dans un autre réacteur de mélange et/ou lors duquel l'activation et/ou la transformation s'effectue par sollicitation mécanique par choc et pression dans une machine de broyage fin.

2. Procédé selon la revendication 1, lors duquel au moins un partenaire réactionnel est activé thermiquement.

3. Procédé selon la revendication 1 ou 2, lors duquel de l'eau apparaissant lors de la transformation est séparée des partenaires réactionnels par évaporation.

4. Procédé selon une des revendications 1 à 3, lors duquel les produits de départ sont amenés sous forme sèche.

5. Procédé selon une des revendications 1 à 4, lors duquel les composés métalliques sont amenés en tant que mélange de particules en vrac et/ou des acides aminés sont amenés en tant que mélange de particules en vrac.

6. Procédé selon la revendication 5, lors duquel la chaleur générée par l'opération du réacteur de mélange est utilisée pour l'activation thermique et/ou pour l'évaporation de l'eau.

7. Procédé selon une des revendications 2 à 6, lors duquel l'activation thermique et/ou l'évaporation est réalisée à une température comprise entre 30 et 150 °C, est de préférence réalisée à une température de 80 à 120 °C.

8. Procédé selon une des revendications 1 à 7, lors duquel la transformation est poursuivie pendant un stockage du produit de réaction en dehors d'un réacteur et/ou lors duquel le produit de réaction contient de l'eau de réaction libre lors du prélèvement du réacteur.

9. Procédé selon une des revendications 1 à 8, lors duquel des acides aminés-chélates du cuivre et/ou du zinc et/ou du manganèse et/ou du fer et/ou du magnésium et/ou du calcium et/ou du nickel et/ou du cobalt sont fabriqués et/ou lors duquel des acides aminés-chélates de la glycine et/ou de la lysine et/ou de la méthionine et/ou d'autres acides aminés et/ou de mélanges d'acides aminés sont fabriqués.

10. Procédé selon une des revendications 1 à 9, lors duquel le rapport massique des oxydes métalliques et/ou des carbonates métalliques et/ou des hydroxydes métalliques et/ou des sulfates métalliques et/ou des chlorures métalliques sur les acides aminés se monte à 1/2 à 1/5.
